# EUROPÄISCHE PATENTSCHRIFT

(11) **EP 3 632 888 B1**
(45) Veröffentlichungstag und Bekanntmachung des Hinweises auf die Patenterteilung: **14.04.2021**
(21) Anmeldenummer: 19200451.3
(22) Anmeldetag: 30.09.2019
(51) Int. Cl.: C07C 45/50, B01J 21/08, B01J 23/46, B01J 31/02, B01J 31/16, B01J 31/18, B01J 35/04, B01J 37/02, B01J 19/24

(54) **VERFAHREN ZUR IN-SITU HERSTELLUNG EINES HYDROFORMYLIERUNGSKATALYSATORSYSTEMS, WELCHES HETEROGENISIERT AUF EINEM SUPPORT VORLIEGT**
PROCESS FOR THE IN-SITU PRODUCTION OF A HYDROFORMYLATION CATALYST SYSTEM WHICH IS HETEROGENIZED ON A VESSEL
PROCÉDÉ DE FABRICATION SUR PLACE D'UN SYSTÈME DE CATALYSEUR D'HYDROFORMYLATION SE TROUVANT HÉTÉROGÉNISÉ SUR UN SUPPORT

(30) Priorität: 05.10.2018 EP 18198790
(43) Veröffentlichungstag der Anmeldung: 08.04.2020
(73) Patentinhaber: Evonik Operations GmbH, 45128 Essen (DE)
(72) Erfinder: Haßelberg, Jennifer, 44139 Dortmund (DE); Franke, Robert, 45772 Marl (DE); Stenger, Frank, 63755 Alzenau (DE); Kreis, Peter, 44227 Dortmund (DE); Hecht, Corinna, 45721 Haltern am See (DE); Kristen, Marc Oliver, 45721 Haltern am See (DE); Ott, Florian, 45657 Recklinghausen (DE)
(74) Vertreter: Evonik Patent Association

(56) Entgegenhaltungen:
- WO-A1-2015/028284
- GB-A- 1 335 531

## Beschreibung

Die vorliegende Erfindung betrifft ein Verfahren zur Herstellung eines Hydroformylierungskatalysators, wobei ein Katalystorsystem, umfassend ein Übergangsmetall, mindestens einen organischen Phosphor-enthaltenden Liganden, einen Stabilisator und optional eine ionische Flüssigkeit, in-situ auf einen Support-Monolith aufgebracht wird, sowie die Verwendung des so hergestellten auf einem Support-Monolith heterogenisierten Katalysatorsystems bei der Hydroformylierung von Olefinen.

Die Hydroformylierung ist mit einer jährlichen globalen Produktionskapazität von mehreren Millionen Tonnen eine der bedeutendsten Reaktionen in der großtechnischen Chemie. Dabei werden Alkene (Olefine) mit einer Mischung aus Kohlenmonoxid und Wasserstoff (auch: Synthesegas oder Syngas) unter Verwendung eines Katalysators zu Aldehyden umgewandelt, die wichtige und wertvolle Zwischenprodukte bei der Herstellung von chemischen Massenprodukten wie Alkoholen, Estern oder Weichmachern sind.

Die Hydroformylierung wird im großtechnischen Maßstab ausschließlich homogen katalysiert durchgeführt. Die löslichen Übergangsmetallkatalysatorsysteme basieren üblicherweise auf Cobalt oder Rhodium, welches oft mit Phosphor-haltigen Liganden, bspw. Phosphinen oder Phosphiten, für die Hydroformylierung von eher kurzkettigen Olefinen eingesetzt wird.

Um Problemen bei der homogen katalysierten Hydroformylierung vorzubeugen, sind Hydroformylierungsverfahren entwickelt worden, bei denen das Katalysatorsystem heterogenisiert wird, insbesondere durch Immobilisierung auf einem Trägermaterial (vgl. einleitende Diskussion in der WO 2015/028284 A1). Die Begriffe Heterogenisierung und Immobilisierung sind demnach so zu verstehen, dass der Katalysator durch Ausbildung eines dünnen Flüssigkeitsfilms mithilfe einer ionischen Flüssigkeit auf der Oberfläche und/oder in den Poren eines festen Trägermaterials immobilisiert wird und keine Reaktionslösung im klassischen Sinne vorliegt, in der der Katalysator homogen gelöst ist.

Im Hinblick auf die Immobilisierung bzw. Heterogenisierung offenbart die bereits erwähnte WO 2015/028284 A1 sogenannte SILP-Systeme (SILP = Supported lonic Liquid Phase), bei der das Katalysatorsystem mit Rhodium, Iridium oder Cobalt als Zentralatom insbesondere auf einem porösen Siliciumdioxid-Träger unter Verwendung einer ionischen Flüssigkeit immobilisiert wird. Der Katalysator wird bei der SILP-Technologie üblicherweise in einer inerten Umgebung (bspw. in einer Glovebox) außerhalb des Reaktors hergestellt und dann als Katalysatorschüttung beispielsweise in einen Festbettreaktor eingefüllt.

Das Problem dabei ist, dass das so hergestellte SILP-Katalysatorsystem nach der Herstellung in einer inerten Umgebung zum Reaktor transportiert und in den Reaktor gefüllt werden muss. Eine solche inerte Umgebung ist während des Transports und dem Einfüllen nur schwer und unter vergleichsweise hohem Kostenaufwand zu realisieren.

Die Aufgabe der vorliegenden Erfindung bestand deshalb darin, ein Verfahren zur Herstellung eines auf einem Support heterogenisierten Hydroformylierungskatalysatorsystems bereitzustellen, welches die vorgenannten Probleme nicht aufweist und insbesondere zu einer Erhöhung des Umsatzes führt.

Gelöst wird diese Aufgabe gemäß Anspruch 1 dadurch, dass das Verfahren zur Herstellung des Hydroformylierungskatalysators in-situ, d.h. direkt im Reaktor, erfolgt und so aufwendige Transport- und Einfüllschritte des gesamten Katalysators unter inerten Bedingungen entfallen.

Ein Gegenstand der vorliegenden Erfindung ist somit ein Verfahren zur in-situ Herstellung eines Hydroformylierungskatalysators, wobei der Hydroformylierungskatalysator ein Katalysatorsystem umfasst, welches heterogenisiert auf einem Support-Monolith vorliegt, wobei das Verfahren zumindest die folgenden Schritte umfasst:
a) Bereitstellen eines Reaktors in dem ein Support-Monolith vorhanden ist und Spülen des Reaktors mit Inertgas;
b) Herstellen einer Katalysatorlösung, welche mindestens einen organischen Phosphor-haltigen Ligand, mindestens ein Metall aus der 8. oder 9. Gruppe des Periodensystems der Elemente, mindestens einen Stabilisator, mindestens ein Lösemittel und optional eine ionische Flüssigkeit enthält;
c) Inkontaktbringen der so hergestellten Katalysatorlösung mit dem Support-Monolith im Reaktor durch Befüllen des Reaktors mit der Katalysatorlösung; und
d) Abtrennen des Lösemittels durch Ablassen aus dem Reaktor und anschließendem Verdampfen der verbliebenen Lösemittelreste durch Einstellung des Drucks und/oder Erhöhung der Temperatur.

Die vorgenannte Schrittabfolge muss nicht wie angegeben exakt chronologisch von a) bis d) durchgeführt werden. Die einzelnen Schritte können auch in anderer Reihenfolge durchgeführt werden. Wichtig ist lediglich, dass die Schritte überhaupt durchgeführt werden. So ist es auch beispielsweise möglich, dass die Bereitstellung des Reaktors mit dem Support-Monolith erst nach Herstellung der Katalysatorlösung erfolgt.

Der Support, auf den das erfindungsgemäße Katalysatorsystem aufgetragen wird, ist ein Monolith, das heißt der Support besteht aus einem Block, ist also ein dreidimensionales Objekt. Der Block kann sowohl einstückig ausgebildet sein als auch aus mehreren, also mindestens zwei Einzelteilen bestehen, die zu dem Block zusammengefügt werden können und/oder miteinander fest oder lösbar verbunden sind. Der Support-Monolith ist aber insbesondere kein Granulat, welches als Katalysatorschüttung in Festbettreaktoren eingesetzt werden kann.

Der Support-Monolith ist vorzugsweise ein sich dreidimensional erstreckendes Bauteil, welches in seinem Querschnitt grundsätzlich beliebige geometrische Formen, beispielsweise rund, eckig, quadratisch o. ä., aufweisen kann. Das sich dreidimensional erstreckende Bauteil, welches als Support-Monolith eingesetzt werden kann, weist in einer bevorzugten Ausführungsform eine Längsrichtung (Richtung der längsten Ausdehnung) in Hauptdurchströmungsrichtung (Richtung in die das Einsatzgemisch und das Synthesegas von Einlass zum Auslass des Reaktors strömen) auf.

Der so geformte Support-Monolith weist zumindest einen durchgängigen Kanal in Hauptdurchströmungsrichtung auf. Der oder die Kanäle können jedoch auch so ausgestaltet sein, dass sie nicht komplett durchgängig sind, sondern zu dem Ende, welches dem Einlass des Reaktors gegenüberliegt, einen Abschluss aufweisen bzw. der Kanal zu diesem Ende hin geschlossen ist. Der Support-Monolith kann auch mindestens zwei oder mehr Kanäle aufweisen. Der Durchmesser der Kanäle kann im Bereich von 0,25 bis 50 mm, vorzugsweise im Bereich von 1 bis 30 mm, weiterhin bevorzugt im Bereich von 1,5 bis 20 mm und besonders bevorzugt im Bereich von 2 bis16 mm liegen. Sind mehrere Kanäle vorhanden, können die Durchmesser der Kanäle gleich oder verschieden voneinander sein. Der Durchmesser der Kanäle ist im Vergleich zu dem oder zu einem der Durchmesser des gesamten Support-Monoliths insbesondere so zu wählen, dass die mechanische Stabilität nicht beeinträchtigt wird.

Darüber kann der Support-Monolith porös sein, also Poren aufweisen. Das erfindungsgemäße Katalysatorsystem kann sich insbesondere in einem festen oder flüssigen Film in diesen Poren befinden. Der Porendurchmesser liegt vorzugsweise im Bereich von 0,9 nm bis 10 µm, weiterhin bevorzugt im Bereich von 10 nm bis 4 µm und besonders bevorzugt im Bereich von 70 nm bis 1 µm. Der Porendurchmesser kann mittels Stickstoffadsorption oder Quecksilber-Porosimetrie gemäß DIN 66133 (Stand: 1993-06) bestimmt werden.

In einer bevorzugten Ausführungsform weist der Support-Monolith zumindest teilweise durchgängige Poren auf, die sich von der Oberfläche zu den Kanälen hin und/oder von einem Kanal zu dem oder zu den nächstliegenden Kanälen erstrecken. Möglich ist auch das mehrere Poren miteinander verbunden sind und so insgesamt eine einzige durchgängige Pore bilden.

Das Katalysatorsystem liegt nach der Herstellung erfindungsgemäß heterogenisiert auf einem Support- Monolith vor. Im Sinne der vorliegenden Erfindung ist der Ausdruck "heterogenisiert auf einem Support-Monolith" so zu verstehen, dass das Katalysatorsystem durch Ausbildung eines dünnen, festen oder flüssigen Films mithilfe des Stabilisators und/oder der ionischen Flüssigkeit auf der inneren und/oder äußeren Oberfläche eines festen Trägermaterials immobilisiert wird. Der Film kann auch bei Raumtemperatur fest und bei Reaktionsbedingungen flüssig sein.

Die innere Oberfläche des festen Trägermaterials umfasst insbesondere die innere Oberfläche der Poren und/oder Kanäle. Immobilisierung umfasst begrifflich sowohl den Fall, dass das Katalysatorsystem und/oder die katalytisch aktive Spezies gelöst in dem festen oder flüssigen Film vorliegen, als auch die Fälle, dass der Stabilisator als Haftvermittler wirkt oder dass das Katalysatorsystem auf der Oberfläche adsorbiert wird, nicht jedoch chemisch bzw. kovalent gebunden auf der Oberfläche vorliegt.

Es liegt erfindungsgemäß also keine Reaktionslösung im klassischen Sinne vor, in der der Katalysator homogen gelöst ist, sondern das Katalysatorsystem befindet sich dispers verteilt auf der Oberfläche und/oder in den Poren des Support-Monoliths.

Der Support-Monolith besteht in einer bevorzugten Ausführungsform der vorliegenden Erfindung aus einem keramischen Material, welches auch die vorgenannten Poren aufweist. Das keramische Material ist vorzugsweise ausgewählt aus der Gruppe, bestehend aus einer Silikatkeramik, einer oxidischen Keramik, einer nitridischen Keramik, einer carbidischen Keramik, einer silicidischen Keramik und Mischungen davon.

Die Silikatkeramik ist vorzugsweise ausgewählt aus Alumosilikat, Magnesiumsilikat und Mischungen davon, wie z. B. Betonit. Die oxidische Keramik ist vorzugsweise ausgewählt aus γ-Aluminiumoxid, α-Aluminiumoxid, Titandioxid, Beryliumoxid, Zirkoniumoxid, Aluminiumtitanat, Bariumtitanat, Zinkoxid, Eisenoxide (Ferrite) und Mischungen davon. Die nitridische Keramik ist vorzugsweise ausgewählt aus Siliciumnitrid, Bornitrid, Siliciumnitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support-Monolith gemäß der vorliegenden Erfindung, auf den das Katalysatorsystem aufgebracht wird, besteht vorzugsweise aus einer carbidischen Keramik.

Auf das keramische Material kann optional zusätzlich ein sogenannter Washcoat aus dem gleichen oder einem unterschiedlichen Keramikmaterial, das für den Support-Monolith verwendet wird, insbesondere ein aus den vorgenannten Keramikmaterialen ausgewähltes Keramikmaterial, vorzugsweise Siliciumoxid, aufgetragen werden. Der Washcoat selber kann porös oder unporös sein, vorzugsweise ist der Washcoat unporös. Die Partikelgröße des Washcoats beträgt vorzugsweise 5 nm bis 3 µm, vorzugsweise 7 nm bis 700nm. Der Washcoat wird verwendet, um die gewünschte Porengröße einzubringen oder zu generieren und/oder um die Oberfläche des Support-Monoliths zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports.

Das erfindungsgemäße Herstellverfahren umfasst in Schritt a) zunächst das Bereitstellen eines Reaktors, in dem der Support-Monolith vorliegt. Der Reaktor ist insbesondere so geformt, dass er den Support-Monolith vollständig aufnehmen kann, vorzugsweise besteht zwischen der Reaktorinnenwand und dem Support ein Spalt. Ein solcher Spalt ist für eine Regenerierung vorteilhaft. Der Reaktor ist dann an den oberen und unteren Enden vorzugsweise mit einer O-RingDichtung abgeschlossen. Der Reaktor ist ansonsten in dem Fachmann bekannter Weise aufgebaut und verfügt beispielsweise über mindestens einen Auslass und mindestens einen Einlass. Ein- und Auslass sind vorzugsweise an sich gegenüberliegenden Seiten angeordnet.

Der Reaktor wird gemäß Schritt a), insbesondere aber vor dem Inkontakbringen mit der Katalysatorlösung in Schritt c), außerdem mit einem Inertgas, beispielsweise Stickstoff, gespült. Das Spülen kann bei 1 bis 25 bar durchgeführt werden, vorzugsweise unter leichtem Überdruck von 20 bis 90 mbar, besonders bevorzugt 30 bis 60 mbar über Normaldruck. Der Reaktor kann vor dem Spülen mit Inertgas abgekühlt werden, um zu verhindern, dass das Lösemittel der einzufüllenden Katalysatorlösung sofort verdampft. Weist das Lösemittel jedoch eine Siedetemperatur auf, die größer ist als die Temperatur des Reaktors kann das Abkühlen des Reaktors entfallen.

Nach dem Spülen mit Inertgas kann der vorhandene Druck, beispielsweise über die Druckregelung, abgelassen werden, vorzugweise bis der Reaktor drucklos ist, also Umgebungsdruck (ca. 1 bar) aufweist. Andererseits kann in dem Reaktor, auch ein Vakuum erzeugt werden, beispielsweise mit einer Vakuumpumpe. In einer Ausgestaltung der vorliegenden Erfindung kann der Reaktor, nach dem Ablassen des Drucks oder nach dem Ziehen des Vakuums erneut mit einem Inertgas wie oben beschrieben gespült werden. Dieser Vorgang von Druck ablassen / Vakuum ziehen und erneutem Spülen kann beliebig oft wiederholt werden.

In Schritt b) wird, insbesondere durch Mischen bei Raumtemperatur und Umgebungsdruck, eine Katalysatorlösung hergestellt, die mindestens einen organischen Phosphor-haltigen Liganden, mindestens ein Übergangsmetall aus der 8. oder 9. Gruppe des Periodensystems der Elemente, beispielsweise in Form eines Chlorids, Oxids, Carboxylats des jeweiligen Übergangsmetalls, mindestens einen Stabilisator und mindestens ein Lösemittel umfasst. Optional kann bei der Herstellung des Katalysatorsystems eine ionische Flüssigkeit verwendet werden, die Katalysatorlösung kann aber auch explizit ohne ionische Flüssigkeit angesetzt werden. Die Herstellung der Katalysatorlösung sollte insbesondere in einer inerten Umgebung, bspw. einer Glovebox erfolgen. Inerte Umgebung heißt in diesem Fall eine möglichst Wasser- und Sauerstofffreie Atmosphäre.

Das Lösemittel kann aus allen Lösemittelklassen gewählt werden (protisch, aprotisch, polar oder unpolar) Voraussetzung für das Lösemittel ist die Löslichkeit von Katalysatorsystem (Ligand, Metall Precursor, Stabilisator und optional die ionische Flüssigkeit) und bevorzugt auch der bei der Hydroformylierung entstehenden Hochsieder. Die Löslichkeit kann innerhalb des Immobilisierungsschrittes durch Aufheizen erhöht werden. Das Lösemittel ist vorzugsweise aprotisch, polar, wie z. B. Acetonitril und Ethylacetat oder aber auch aprotisch, unpolar wie z. B. THF und Dietehylether. Auch Chlorkohlenwasserstoffe wie z. B. Dichlormethan, können als Lösemittel verwendet werden.

Das Übergangsmetall aus der 8. oder 9. Gruppe des Periodensystems der Elemente, ist vorzugweise Eisen, Ruthenium, Iridium, Cobalt oder Rhodium, besonders bevorzugt Cobalt und Rhodium.

Der organische Phosphor-enthaltende Ligand zum Einsatz bei der Herstellung der erfindungsgemäßen Katalysatorlösung weist vorzugsweise die allgemeine Formel (VI)

R'-A- R"-A- R'" (VI)

wobei R', R" und R'" jeweils organische Reste sind, mit der Maßgabe das R' und R'" nicht identisch sind, und beide A jeweils eine brückende -O-P(-O)₂-Gruppe sind, wobei zwei der drei Sauerstoffatome -O-jeweils an Rest R' und den Rest R'" gebunden sind. Die organischen Reste R', R" und R'" enthalten vorzugsweise keine endständige Trialkoxysilangruppe.

In einer bevorzugten Ausführungsform sind R', R" und R'" in der Verbindung der Formel (VI), vorzugsweise ausgewählt aus substituierten oder unsubstituierten 1,1-Biphenyl-, 1,1'-Binaphthyl- und ortho-Phenylgruppen, insbesondere aus substituierten oder unsubstituierten 1,1'-Biphenylgruppen, mit der Maßgabe das R' und R'" nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Der Stabilisator ist vorzugsweise eine organische Aminverbindung, besonders bevorzugt eine organische Aminverbindung, die mindestens eine 2,2,6,6-Tetramethylpiperidineinheit nach Formel (I) enthält:

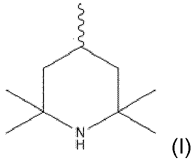

In einer besonders bevorzugten Ausführungsform der vorliegenden Erfindung ist der Stabilisator ausgewählt aus der Gruppe, bestehend aus den Verbindungen der nachfolgenden Formeln (l.1), (I.2), (I.3), (I.4), (I.5), (I.6), (I.7) und (I.8). 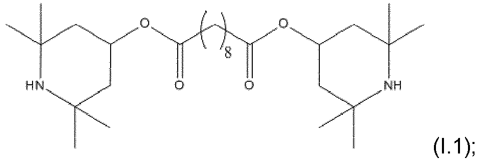 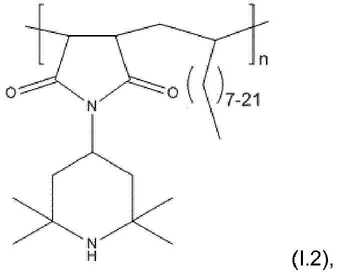 wobei n einer ganzen Zahl von 1 bis 20 entspricht; 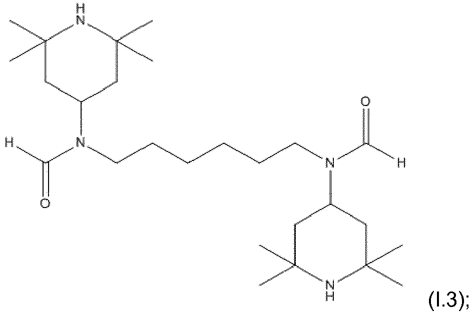 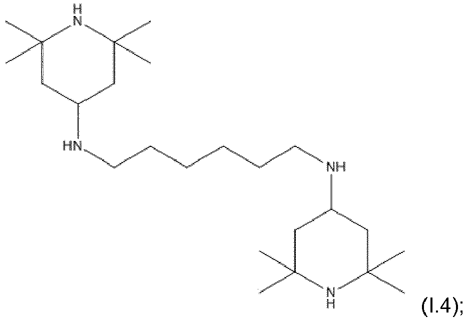 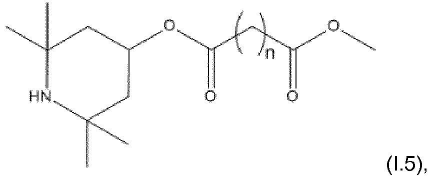 wobei n einer ganzen Zahl von 1 bis 12 entspricht; 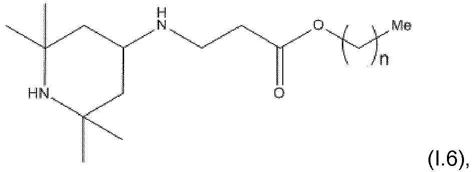 wobei n einer ganzen Zahl von 1 bis 17 entspricht; 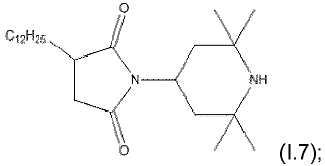 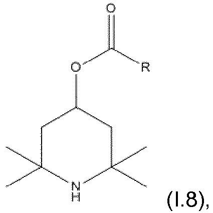 wobei R einer C6- bis C20-Alkylgruppe entspricht.

Die optional vorliegende ionische Flüssigkeit im Sinne der vorliegenden Erfindung ist eine nahezu wasserfrei (Wassergehalt < 1,5 Gew.-% bezogen auf die gesamte ionische Flüssigkeit) Flüssigkeit, die bei Normaldruck (1,01325 bar) und vorzugsweise bei 25 °C flüssig ist. Die ionische Flüssigkeit besteht vorzugsweise zu mehr als 98 Gew.-% aus Ionen.

In einer bevorzugten Ausführungsform wird das Anion der ionischen Flüssigkeit ausgewählt aus der Gruppe, bestehend aus Tetrafluoroborat [BF4]⁻; Hexafluorophosphat [PF6]⁻; Dicyanamid [N(CN)_{2]}⁻; Bis(trifluoromethylsulfonyl)imid [NTf_{2]}⁻; Tricyanomethid [C(CN)_{3]}⁻; Tetracyanoborat [B(CN)_{4]}⁻; Halogeniden, insbesondere Cl⁻, Br⁻, F⁻, I⁻; Hexafluoroantimonat [SbF₆]⁻; Hexafluoroarsenat [AsF₆]⁻; Sulfat [SO₄]²⁻; Tosylat [C₇H₇SO₃]⁻; Triflat CF₃SO₃⁻; Nonaflat [C₄F₉SO₃]⁻; Tris-(Pentafluoroethyl)-trifluorophosphat [PF₃(C₂F₅)₃]⁻; Thiocyanat [SCN]⁻; Carbonat [CO₃]²⁻; [RA-COO]⁻; [RA-SO₃]⁻; [RA-SO₄]⁻; [RAPO₄RB]- und [(RA-SO₂)₂N]⁻, wobei RA und RB gleich oder ungleich voneinander sein können und jeweils eine lineare oder verzweigte aliphatischer oder alicyclischer Alkylgruppe mit 1 bis 12 Kohlenstofatomen, eine Perfluoralkylgruppe oder eine C5-C18-substituierte Arylgruppe ist, die durch ein oder mehrere Halogenatome substituiert ein kann.

Das Kation der ionischen Flüssigkeit wird vorzugsweise ausgewählt aus der Gruppe, bestehend aus quaternäre Ammonium-Kationen der allgemeinen Formel [NR¹R²R³R⁴]⁺ wobei R¹, R², R³, R⁴ jeweils unabhängig voneinander eine C1-C8-Alkylgruppe darstellen; Phosphonium-Kationen der allgemeinen Formel [PR¹R²R³R⁴]⁺ wobei R¹, R², R³, R⁴ jeweils unabhängig voneinander eine C1-C8-Alkylgruppe darstellen; Imidazolium-Kationen der allgemeinen Formel (II) 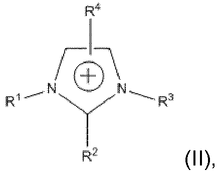 wobei R¹, R², R³ und R⁴ jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Pyridinium-Kationen der allgemeinen Formel (III) 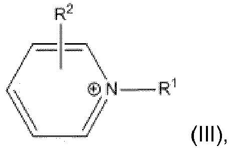 wobei R¹ und R² jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Pyrazolium-Kationen der allgemeinen Formel (IV) 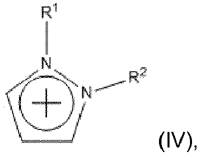 wobei R¹ und R² jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Triazolium-Kationen der allgemeinen Formel (V) 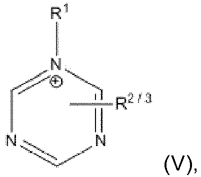 wobei R¹ und R² und/oder R³ jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen.

In einer bevorzugten Ausführungsform ist das Kation der ionischen Flüssigkeit ein Imidazolium-Kation nach der vorgenannten allgemeinen Formel (II) mit entsprechender Definition der Reste R¹ bis R⁴. In einer besonders bevorzugten Ausführungsform ist die ionische Flüssigkeit ausgewählt aus der Gruppe, bestehend aus 1-Ethyl-3-methylimidazolium bis(trifluoromethylsulfonyl)imid, 1-Butyl-3-methylimidazolium hexafluorophosphat, 1-Butyl-3-methylimidazolium tetrafluoroborat, 1-Butyl-3-methylimidazolium bis(trifluoromethylsulfonyl)imid, 1-Ethyl-3-methylimidazolium ethylsulfat, Trioctyl-Methylammonium bis(trifluoromethylsulfonyl)imid und 1-Butyl-3-methylimidazolium octylsulfat.

Die optional vorhandene ionische Flüssigkeit kann als Trägerlösung für den Übergangsmetallkatalysator mit Liganden und den Stabilisator dienen. Dabei ist es wichtig, dass die ionische Flüssigkeit die Reaktanden (Einsatzolefine und Synthesegas) in ausreichendem Maße aufnehmen, d. h. lösen, kann und einen vergleichsweise niedrigen Dampfdruck aufweist, damit das Katalysatorsystem auch bei hohen Temperaturen als Flüssigkeitsreservoir vorliegt. Es konnte jedoch überraschenderweise herausgefunden werden, dass auch der Stabilisator einen stabilen Flüssigkeitsfilm in den Poren des Support-Monoliths ausbilden kann und damit in der Lage ist, die ionische Flüssigkeit teilweise oder vollständig zu ersetzen.

Für alle filmbildenden Komponenten, d. h. in diesem Fall die ionische Flüssigkeit und/oder der Stabilisator, sollte die Gaslöslichkeit für die Reaktanden besser sein als die Gaslöslichkeit der Produkte. Bereits dadurch kann eine partielle Stofftrennung zwischen eingesetzten Eduktolefinen und gebildeten Produktaldehyden erreicht werden. Grundsätzlich wären dafür auch andere filmbildende Substanzen denkbar, allerdings ist darauf zu achten, dass es nicht zu einer erhöhten Hochsiederbildung kommt und/oder die Nachführung der Eduktolefine eingeschränkt wird.

Die so hergestellte Katalysatorlösung wird dann in Schritt c) mit dem Support-Monolith (optional inkl. Washcoat) durch Ein- bzw. Befüllen in den Reaktor in Kontakt gebracht (sog. in-situ-Imprägnierung). Dazu kann die Katalysatorlösung vor dem Befüllen des Reaktors in einem Druckgefäß vorgelegt und vorzugsweise mit einem Inertgasüberdruck von 1 bis 25 bar, besonders bevorzugt einem leichten Inertgasüberdruck von 20 bis 90 mbar, vorzugsweise 30 bis 60 mbar über Reaktordruck beaufschlagt werden. Das Inertgas kann ein Edelgas, ein Alkan, beispielsweise Butan, oder Stickstoff sein. Die Katalysatorlösung kann dann mit dem genannten Überdruck, mit dem das Druckgefäß beaufschlagt ist, insbesondere druckgetrieben in den Reaktor eingefüllt werden. Der Druck des Druckgefäßes sollte beim Befüllen höher sein als im Reaktor. Dabei können Temperaturen im Bereich von 20 bis 150 °C vorliegen. Der Druck im Reaktor kann beim Befüllen 1 bis 25 bar betragen. Herrscht ein Druck im Reaktor, der höher ist als der Normaldruck, muss das Einfüllen mit einem noch höheren Druck eingefüllt werden.

Eine andere Möglichkeit des Befüllens ist, dass der Reaktor nach dem Spülen mit Inertgas im Vakuum gehalten wird und die Katalysatorlösung durch den Unterdruck in den Reaktor gezogen wird. Dabei sollte für die Herstellung der Katalysatorlösung ein Lösemittel verwendet werden, welches bei dem herrschenden Vakuum bzw. Unterdruck und den herrschenden Temperaturen siedet.

Das Befüllen des Reaktors mit der Katalysatorlösung kann über den normalen Ein- bzw. Auslass erfolgen. Flüssigkeitsverteiler oder Düsen innerhalb des Reaktors können für eine gleichmäßige Verteilung der Katalysatorflüssigkeit sorgen, ebenso wie optional vorliegende Druckverlusteinbauten oder Regelungen für die Dosiergeschwindigkeit.

Nach dem Aufbringen des Katalysatorsystems wird das Lösemittel in Schritt d) abgetrennt. Dabei wird zunächst die restliche Katalysatorlösung über den Ein- oder Auslass des Reaktors abgelassen. Danach werden im Reaktor verbliebene Lösemittelreste durch Einstellen des Drucks oder Erhöhung der Temperatur verdampft. Die Einstellung des Drucks kann in einer anderen Ausführungsform auch unter gleichzeitiger Erhöhung der Temperatur durchgeführt werden. Die Temperatur kann in Abhängigkeit vom Lösemittel 20 bis 150 °C betragen, wobei die Temperatur beim Verdampfen, gleiche Druckbedingungen vorausgesetzt, höher ist als beim Einfüllen in Schritt c). Der Siedepunkt einer chemischen Verbindung ist bekanntermaßen druckabhängig, wobei bei geringen Drücken auch geringere Temperaturen ausreichen, damit die Verbindung verdampft. Herrscht daher in Schritt d) ein geringerer Druck als in Schritt c) kann die Temperatur auch geringer sein als in Schritt c). Der Druck kann in Abhängigkeit vom Lösemittel auf ein Hochvakuum (10⁻³ bis 10⁻⁷ mbar) eingestellt werden, je nach Lösemittel und Temperatur sind aber auch Überdrücke von einigen mbar bis zu mehreren bar denkbar.

Der Stabilisator und die optional vorhandene ionische Flüssigkeit verbleiben mit dem Katalysator aus dem Übergangsmetall, insbesondere Cobalt oder Rhodium, und dem organischen Phosphor-haltigen Liganden heterogenisiert auf dem Support-Monolith.

Die beanspruchte Vorgehensweise für die Herstellung des Hydroformylierungskatalysators hat auch Vorteil, dass keine zeitaufwändigen Ein- und Ausbauschritte notwendig sind, die einen längeren Ausfall des Reaktors zur Folge hätten. Zudem ist die Größe des Support-Monoliths dann nicht mehr dadurch limitiert, dass geeignete Räumlichkeiten mit inerten Umgebungen einer bestimmten Größe vorliegen. Die Größe des Support-Monoliths kann in Abhängigkeit vom Reaktordesign frei gewählt werden.

Nach erfolgtem Aufbringen des Katalysatorsystems auf den Support-Monolith und erfolgter Abtrennung des Lösemittels kann die Anlage, insbesondere der Reaktor durch eine zwei- oder mehrstufige Anfahrprozedur hochgefahren werden, also in den Betrieb überführt werden. Der so hergestellte Hydroformylierungskatalysator kann insbesondere für die Hydroformylierung von C2-bis C8-Olefinen verwendet werden.

Als Einsatzgemisch für die Hydroformylierung können alle Gemische eingesetzt werden, die C2-bis C8-Olefine, vorzugsweise C2- bis C5-Olefine, insbesondere Ethen, Propen, 1-Buten, 2-Buten, 1-Penten oder 2-Penten, als Edukte umfassen. Die Menge an Olefinen in den Einsatzgemischen sollte verständlicherweise hoch genug sein, um eine Hydroformylierungsreaktion wirtschaftlich betreiben zu können. Dazu gehören insbesondere technische Gemische der petrochemischen Industrie, wie beispielsweise Raffinatströme (Raffinat I, II oder III) oder Rohbutan. Rohbutan umfasst gemäß der vorliegenden Erfindung 5 bis 40 Gew.-% Butene, vorzugsweise 20 bis 40 Gew.-% Butene (die Butene setzen sich zusammen aus 1 bis 20 Gew.-% 1-Buten und 80 bis 99 Gew.-% 2-Buten) und 60 bis 95 Gew.-% Butane, vorzugsweise 60 bis 80 Gew.-% Butane.

Die Hydroformylierung wird vorzugsweise bei den folgenden Bedingungen durchgeführt: Die Temperatur bei der Hydroformylierung sollte im Bereich von 65 bis 200 °C, vorzugsweise 75 bis 175 °C und besonders bevorzugt 85 bis 150 °C liegen. Der Druck sollte 35 bar, vorzugsweise 30 bar, besonders bevorzugt 25 bar während der Hydroformylierung nicht überschreiten. Das molare Verhältnis zwischen Synthesegas und dem zweiten Einsatzgemisch sollte zwischen 6:1 und 1:1, vorzugsweise zwischen 5:1 und 3:1 liegen.

Mit fortschreitender Betriebszeit kann die Aktivität des Hydroformylierungsatalysators abnehmen, beispielsweise durch die Anreicherung von Hochsiedern und/oder der Belegung oder Desaktivierung von aktiven Zentren. Die Hochsieder können zu einer verstärkten Kondensation in den Poren führen, sodass die Poren nicht mehr oder langsamer für die Eduktolefine zugänglich sind. Andererseits können einige Nebenprodukte zu einem Zerfall des Katalysatorsystems führen, wodurch die Aktivität des Katalysators ebenfalls abnimmt. Eine Abnahme der Katalysatoraktivität kann beispielsweise anhand des Absinkens von Umsätzen oder Selektivitäten, insbesondere durch eine entsprechende Analytik mittels Ramanspektroskopie, Gaschromatographie oder Massendurchflussmesser (MDM) ermittelt werden. Möglich wäre auch ein modellbasiertes Überwachen der Katalysatoraktivität. Dies würde eine von den Betriebsbedingungen unabhängige Methode zur Überwachung der Katalysatoraktivität darstellen, aber auch um den Verlauf zu extrapolieren und damit eine Revisions-/Regenerationsplanung zu unterstützen.

In dem Fall einer unzureichenden Katalysatoraktivität kann das Katalysatorsystem, welches heterogenisiert auf dem porösen keramischen Support vorliegt, ausgetauscht werden. Dazu kann der Reaktor bzw. der Support in dem Reaktor einmalig oder mehrmalig mit einem Lösemittel gespült werden. Durch das Spülen kann das Katalysatorsystem demobilisiert und entfernt werden. Das Lösemittel kann eines der für die Herstellung der Katalysatorlösung genannten Lösemittel sein. Die Temperatur beim Spülen mit Lösemittel kann 20 bis 150 °C betragen. Der Druck kann beim Spülen mit Lösemittel zudem 1 bis 25 bar betragen.

Nach dem Spülen wird der Support ein- oder mehrfach neu imprägniert, insbesondere mit der vorher beschriebenen in-situ-Imprägnierung des Supports. Die in-situ-Imprägnierung wird damit erneuert und das heterogenisierte Katalysatorsystem frisch aufgebracht. Die erneute in-situ-Imprägnierung kann unter genau den gleichen Bedingungen durchgeführt werden, wie sie für die erste in-situ-Imprägnierung oben beschrieben worden sind.

Aufgrund der Tatsache, dass das Katalysatorsystem durch Spülen und erneutes Aufbringen vollständig ausgetauscht wird, können diese Schritte ständig wiederholt werden, sobald die Aktivität des Katalysators erneut absinkt. Ein weiterer Vorteil ist, dass sowohl Hochsieder und Produktaldehyde als auch Zerfallsprodukte des Katalysatorsystems ausgeschleust werden können. Es sollte jedoch darauf geachtet werden, dass die Eigenschaften des Supports durch Demobilisierung und erneute in-situ-Imprägnierung nicht beeinträchtigt werden. Andernfalls wäre auch ein Austausch des porösen keramischen Supports durchzuführen.

Eine weitere Option ist, dass der gesamte poröse keramische Support, auf dem das Katalysatorsystem heterogenisiert vorliegt, ausgetauscht wird. Das Katalysatorsystem, welches auf dem (aus dem Reaktor entfernten) Support heterogenisierten vorliegt, kann dann außerhalb des Reaktors wie oben beschrieben ausgetauscht und bis zum nächsten Einbau und Einsatz im Reaktor gelagert werden. Wie erwähnt ist beim Auftrag des Katalysatorsystems eine inerte Umgebung erforderlich, weshalb die Handhabung und Lagerung bei der genannten Verfahrensweise von Aus- und Einbau des Supports unter entsprechenden Bedingungen erfolgen sollte.

Grundsätzlich ist eine Verfahrensführung bevorzugt bei der in der zweiten Reaktionszone mehrere Reaktoren parallel vorliegen und diese alternierend verwendet werden. Dabei wird mindestens ein Reaktor (a) für die Hydroformylierung eingesetzt, also einer, der in Betrieb ist, und mindestens ein Reaktor (b), der in Wartehaltung ist. Das ist so zu verstehen, dass sobald bei dem sich in Betrieb befindende Reaktor (a) eine nicht mehr ausreichende Katalysatoraktivität festgestellt wird, der Strom des zweiten Einsatzgemisches von diesem Reaktor (a) auf den nächsten Reaktor (b) in Wartehaltung, der damit in Betrieb genommen wird, umgeschaltet wird. Reaktor (a) wird dann in Regenerationsmodus überführt, wo das Katalysatorsystem wie oben beschrieben regeneriert oder demobilisiert und der Support-Monolith neu imprägniert wird, und dann in die Warteposition überführt bis ein erneuter Wechsel mit Reaktor (b) erfolgt. Dies Prinzip lässt sich auch auf 3 oder mehr Reaktoren anwenden, wobei ein Reaktor in Betrieb ist, einer oder mehre Reaktoren gleichzeitig in Wartehaltung sind und einer oder mehrere Reaktoren gleichzeitig im Regenerationsmodus sind.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand eines Beispiels näher erläutert. Alternative Ausführungsformen der vorliegenden Erfindung sind in analoger Weise erhältlich.

### Beispiel:

### Versuch 1: Herstellung und Untersuchung eines erfindungsgemäßen Katalysatorsystems

Als Support wurde ein Monolith aus Siliciumcarbid mit einer Länge von ca. 20 cm und einem Durchmesser von ca. 25 mm verwendet. Der Support war porös und wurde mit einem Washcoat (SiO₂) vorbehandelt. Der Support wies 31 Kanäle mit einem Durchmesser von ca. 3 mm auf. Der Support wurde in einen Reaktor eingesetzt und mit einer Katalysatorlösung, enthaltend Rh(acac)(CO)₂, Biphephos (Ligand), Bis(2,2,6,6-tetramethyl-4-piperidyl)sebacat (Stabilisator) und Dichlormethan als Lösemittel und hergestellt durch Mischen in einer inerten Umgebung (Glovebox), beaufschlagt. Dazu wurde die Katalysatorlösung nach dem Spülen des Reaktors mit Stickstoff mit leichtem Überdruck in den Reaktor eingeleitet. Nach Entfernen des Lösemittels aus dem Reaktor durch Ablassen und Verdampfen wurde das auf dem Support heterogenisierte Katalysatorsystem zur Hydroformylierung verwendet.

Als Einsatzgemisch wurde ein Kohlenwasserstoffstrom mit der folgenden Zusammensetzung verwendet:

| | Menge (Gew.-%) |
|---|---|
| 1-Buten / iso-Buten | 19,14 |
| cis-2-Buten | 19,10 |
| trans-2-Buten | 28,40 |
| n-Butan | 30,80 |
| iso-Butan | 0,02 |
| 2-Methyl-butan | 2,50 |

Das Einsatzgemisch wurde zusammen mit Synthesegas (molares Verhältnis Synthesegas :
Einsatzgemisch = 3,5 : 1) zur Hydroformylierung mit einem Gasvolumenstrom von 390 ml/min in den Reaktor geleitet. Die Hydroformylierung wurde bei einer Temperatur von 120 °C und einem Druck von 10 bar durchgeführt. Der Gesamtumsatz an Butenen (also der Umsatz aller im Einsatzgemisch befindlichen Butene) und die n/iso-Selektivität (Verhältnis von linearen zu verzweigten Produkten) wurde gaschromatographisch über die Produktzusammensetzung ermittelt.

Nach einer Versuchsdauer von 500 Stunden betrug der Gesamtumsatz an Butenen 45% und die n/iso-Selektivität 97%.

Nach einer Betriebsdauer von ca. 3500 Stunden wurde das Katalysatorsystem durch eine erneute in-situ-Imprägnierung erneuert und ein weiterer Versuch durchgeführt. Das Einsatzgemisch hatte die oben genannte Zusammensetzung. Die Versuchsbedingungen waren identisch zu den Versuchsbedingungen im ersten Durchlauf (Gasvolumenstrom = 390 ml/min / T = 120 °C / Druck = 10 bar). Nach einer Versuchsdauer von 500 Stunden nach dem Erneuern des Katalysatorsystems auf dem Support betrug der Gesamtumsatz an Butenen 35% und die n/iso-Selektivität 97%.

Es konnte also gezeigt werden, dass die in-situ-Imprägnierung wiederholt werden kann und der Umsatz sowie die n/iso-Selektivität ausreichend hoch sind.

## Patentansprüche

1. Verfahren zur in-situ Herstellung eines Hydroformylierungskatalysators, wobei der Hydroformylierungskatalysator ein Katalysatorsystem umfasst, welches heterogenisiert auf einem Support-Monolith vorliegt, wobei das Verfahren zumindest die folgenden Schritte umfasst:
a) Bereitstellen eines Reaktors in dem ein Support-Monolith vorhanden ist, wobei der Support-Monolith zumindest einen durchgängigen Kanal in Hauptdurchströmungsrichtung aufweist, und Spülen des Reaktors mit Inertgas;
b) Herstellen einer Katalysatorlösung, welche mindestens einen organischen Phosphor-haltigen Ligand, mindestens ein Metall aus der 8. oder 9. Gruppe des Periodensystems der Elemente, mindestens einen Stabilisator, mindestens ein Lösemittel und optional eine ionische Flüssigkeit enthält;
c) Inkontaktbringen der so hergestellten Katalysatorlösung mit dem Support-Monolith im Reaktor durch Befüllen des Reaktors mit der Katalysatorlösung; und
d) Abtrennen des Lösemittels durch Ablassen aus dem Reaktor und anschließendem Verdampfen der verbliebenen Lösemittelreste durch Einstellung des Drucks und/oder Erhöhung der Temperatur.

2. Verfahren nach Anspruch 1, wobei das Spülen des Reaktors mit Inertgas in Schritt a) mit einem Druck von 1 bis 25 bar, vorzugsweise unter leichtem Überdruck von 20 bis 90 mbar, besonders bevorzugt von 30 bis 60 mbar erfolgt.

3. Verfahren nach Anspruch 1 oder 2, wobei der Druck im Reaktor nach dem Spülen mit Inertgas gemäß Schritt a) verringert wird, vorzugsweise so lange bis der Reaktor drucklos (gegenüber Normaldruck) ist.

4. Verfahren nach einem der Ansprüche 1 bis 3, wobei die Katalysatorlösung für das Befüllen des Reaktors in Schritt c) vorher in einem Druckgefäß vorgelegt und vorzugsweise mit einem Inertgasüberdruck von 1 bis 25 bar, vorzugsweise von 20 bis 90 mbar beaufschlagt wird.

5. Verfahren nach einem der Ansprüche 1 bis 4, wobei die Katalysatorlösung in Schritt c) mit dem genannten Inertgasüberdruck in den Reaktor eingefüllt wird.

6. Verfahren nach einem der Ansprüche 1 bis 4, wobei beim Einfüllen der Katalysatorlösung in Schritt c) Temperaturen im Bereich von 20 bis 150 °C vorliegen.

7. Verfahren nach einem der Ansprüche 1 bis 6, wobei beim Verdampfen der verbliebenen Lösemittelreste eine Temperatur im Bereich von 20 bis 150°C vorliegt wobei die Temperatur beim Verdampfen, gleiche Druckbedingungen vorausgesetzt, höher ist als beim Einfüllen in Schritt c).

8. Verfahren nach einem der Ansprüche 1 bis 7, wobei der Support-Monolith aus einem porösen keramischen Material besteht, welches aus der Gruppe, bestehend aus einer Silikatkeramik, einer oxidischen Keramik, einer nitridischen Keramik, einer carbidischen Keramik, einer silicidischen Keramik und Mischungen davon, ausgewählt wird.

9. Verfahren nach Anspruch 8, wobei das poröse keramische Material, aus dem der Support-Monolith besteht, eine carbidische Keramik ist.

10. Verfahren nach einem der Ansprüche 1 bis 9, wobei der Support-Monolith einen oder mehrere, vorzugsweise durchgängige Kanäle in Hauptdurchströmungsrichtung aufweist.

11. Verfahren nach einem der Ansprüche 1 bis 10, wobei das Metall in der Katalysatorlösung in Schritt b) Eisen, Iridium, Cobalt oder Rhodium ist, vorzugsweise Cobalt oder Rhodium.

12. Verwendung eines Hydroformylierungskatalysators, hergestellt nach einem der Ansprüche 1 bis 11, zur Hydroformylierung von C2- bis C8-Olefinen.

13. Verwendung nach Anspruch 12, wobei die Hydroformylierung bei einer Temperatur im Bereich von 65 bis 200 °C, vorzugsweise 75 bis 175 °C und besonders bevorzugt 85 bis 150 °C, durchgeführt wird.

14. Verwendung nach Anspruch 12 oder 13, wobei der Druck bei der Hydroformylierung nicht größer als 35 bar, vorzugsweise nicht größer als 30 bar, besonders bevorzugt nicht größer als 25 bar ist.

## Claims

1. Process for the in-situ production of a hydroformylation catalyst, wherein the hydroformylation catalyst comprises a catalyst system which is heterogenized on a support monolith, wherein the process comprises at least the following steps:
a) provision of a reactor in which a support monolith is present, wherein the support monolith has at least one through-channel in the main flow direction, and flushing of the reactor with inert gas;
b) production of a catalyst solution which contains at least one organic phosphorus-containing ligand, at least one metal of group 8 or 9 of the Periodic Table of the Elements, at least one stabilizer, at least one solvent and optionally an ionic liquid;
c) contacting of the catalyst solution produced in this way with the support monolith in the reactor by filling of the reactor with the catalyst solution; and
d) removal of the solvent by drainage from the reactor and subsequent evaporation of the remaining solvent residues by adjustment of the pressure and/or increasing the temperature.

2. Process according to Claim 1, wherein the flushing of the reactor with inert gas in step a) is carried out at a pressure of from 1 to 25 bar, preferably under a slight gauge pressure of from 20 to 90 mbar, particularly preferably from 30 to 60 mbar.

3. Process according to Claim 1 or 2, wherein the pressure in the reactor is reduced after the flushing with inert gas in step a), preferably until the reactor is unpressurized (relative to atmospheric pressure).

4. Process according to any of Claims 1 to 3, wherein the catalyst solution for the filling of the reactor in step c) is placed in a pressure vessel beforehand and preferably subjected to an inert gas gauge pressure of from 1 to 25 bar, preferably from 20 to 90 mbar.

5. Process according to any of Claims 1 to 4, wherein the catalyst solution is introduced with the specified inert gas gauge pressure into the reactor in step c).

6. Process according to any of Claims 1 to 4, wherein temperatures in the range from 20 to 150°C prevail during introduction of the catalyst solution in step c).

7. Process according to any of Claims 1 to 6, wherein a temperature in the range from 20 to 150°C prevails during evaporation of the remaining solvent residues, where the temperature during evaporation is, providing the same pressure conditions, greater than during the introduction in step c).

8. Process according to any of Claims 1 to 7, wherein the support monolith consists of a porous ceramic material which is selected from the group consisting of a silicate ceramic, an oxidic ceramic, a nitridic ceramic, a carbidic ceramic, a silicidic ceramic and mixtures thereof.

9. Process according to Claim 8, wherein the porous ceramic material of which the support monolith consists is a carbidic ceramic.

10. Process according to any of Claims 1 to 9, wherein the support monolith has one or more channels, preferably through-channels, in the main flow direction.

11. Process according to any of Claims 1 to 10, wherein the metal in the catalyst solution in step b) is iron, iridium, cobalt or rhodium, preferably cobalt or rhodium.

12. Use of a hydroformylation catalyst produced according to any of Claims 1 to 11 for the hydroformylation of C2-C8 olefins.

13. Use according to Claim 12, wherein the hydroformylation is carried out at a temperature in the range from 65 to 200°C, preferably from 75 to 175°C and particularly preferably from 85 to 150°C.

14. Use according to Claim 12 or 13, wherein the pressure in the hydroformylation is not greater than 35 bar, preferably not greater than 30 bar, particularly preferably not greater than 25 bar.

## Revendications

1. Procédé pour la préparation *in situ* d'un catalyseur d'hydroformylation, le catalyseur d'hydroformylation comprenant un système de catalyseur qui est présent de manière hétérogénéisée sur un monolithe de support, le procédé comprenant au moins les étapes suivantes :
a) mise à disposition d'un réacteur dans lequel un monolithe de support est présent, le monolithe de support présentant au moins un canal continu dans la direction d'écoulement principal, et rinçage du réacteur avec un gaz inerte ;
b) préparation d'une solution de catalyseur, qui contient au moins un ligand organique contenant du phosphore, au moins un métal du groupe 8 ou 9 du système périodique des éléments, au moins un stabilisateur, au moins un solvant et éventuellement un liquide ionique ;
c) mise en contact de la solution de catalyseur ainsi préparée avec le monolithe de support dans le réacteur par chargement du réacteur avec la solution de catalyseur ; et
d) séparation du solvant par évacuation du réacteur et évaporation ultérieure des résidus restants de solvant par l'ajustement de la pression et/ou l'augmentation de la température.

2. Procédé selon la revendication 1, le rinçage du réacteur avec un gaz inerte dans l'étape a) étant réalisé avec une pression de 1 à 25 bar(s), de préférence sous une légère surpression de 20 à 90 mbars, particulièrement préférablement de 30 à 60 mbars.

3. Procédé selon la revendication 1 ou 2, la pression dans le réacteur après le rinçage avec un gaz inerte selon l'étape a) étant réduite, de préférence jusqu'à ce que le réacteur soit sans pression (par rapport à la pression normale).

4. Procédé selon l'une quelconque des revendications 1 à 3, la solution de catalyseur pour la charge du réacteur dans l'étape c) étant placée dans un récipient sous pression et de préférence soumise à une surpression de gaz inerte de 1 à 25 bar(s), de préférence de 20 à 90 mbars.

5. Procédé selon l'une quelconque des revendications 1 à 4, la solution de catalyseur dans l'étape c) étant introduite dans le réacteur avec la surpression de gaz inerte mentionnée.

6. Procédé selon l'une quelconque des revendications 1 à 4, dans lequel lors de l'introduction de la solution de catalyseur dans l'étape c), des températures dans la plage de 20 à 150 °C sont présentes.

7. Procédé selon l'une quelconque des revendications 1 à 6, dans lequel lors de l'évaporation des résidus restants de solvant, une température dans la plage de 20 à 150 °C est présente, la température lors de l'évaporation étant supérieure à celle lors de l'introduction dans l'étape c), à conditions de pression égales.

8. Procédé selon l'une quelconque des revendications 1 à 7, le monolithe de support étant constitué d'un matériau céramique poreux qui est choisi dans le groupe constitué par une céramique de silicate, une céramique oxydée, une céramique nitrurée, une céramique carburée, une céramique siliciurée et des mélanges correspondants.

9. Procédé selon la revendication 8, le matériau céramique poreux, qui constitue le monolithe de support, étant une céramique carburée.

10. Procédé selon l'une quelconque des revendications 1 à 9, le monolithe de support présentant un ou plusieurs canaux, de préférence continus, dans la direction d'écoulement principal.

11. Procédé selon l'une quelconque des revendications 1 à 10, le métal dans la solution de catalyseur dans l'étape b) étant le fer, l'iridium, le cobalt ou le rhodium, de préférence le cobalt ou le rhodium.

12. Utilisation d'un catalyseur d'hydroformylation, préparé selon l'une quelconque des revendications 1 à 11, pour l'hydroformylation d'oléfines en C₂₋₈.

13. Utilisation selon la revendication 12, l'hydroformylation étant mise en œuvre à une température dans la plage de 65 à 200 °C, de préférence 75 à 175 °C et particulièrement préférablement 85 à 150 °C.

14. Utilisation selon la revendication 12 ou 13, la pression lors de l'hydroformylation n'étant pas supérieure à 35 bars, de préférence pas supérieure à 30 bars, particulièrement préférablement pas supérieure à 25 bars.
